# EUROPEAN PATENT SPECIFICATION

(11) **EP 1 984 805 B1**
(45) Date of publication and mention of the grant of the patent: **16.06.2010**
(21) Application number: 07701844.8
(22) Date of filing: 30.01.2007
(51) Int. Cl.: G06F 3/033, A61M 16/00, A61B 5/08

(54) **A method and a device for simplifying a diagnostic assessment of a mechanically ventilated patient**
Verfahren und Einrichtung zum Vereinfachen einer diagnostischen Bewertung eines mechanisch beatmeten Patienten
Procedé et dispositif de simplification d'une évaluation diagnostique d'un patient mécaniquement ventilé

(30) Priority: 30.01.2006 CH 147062006; 07.11.2006 CH 17652006
(43) Date of publication of application: 29.10.2008
(73) Proprietor: Hamilton Medical AG, 7402 Bonaduz (CH)
(72) Inventor: BRUNNER, Josef, CH-7000 Chur (CH); WYSOCKI, Marc, F-78460 Chevreves (FR); METTIER, Ivo, CH-7012 Felsberg (CH)
(74) Representative: Hasler, Erich
(86) International application number: PCT/CH2007/000041
(87) International publication number: WO 2007/085109

(56) References cited:
- EP-A1- 0 667 168
- GB-A- 2 368 124
- NL-A- 8 801 322
- US-A- 5 915 380
- US-A- 5 921 920
- US-A- 5 931 160
- WACHTER S B ET AL: "Evaluation of a pulmonary graphical display in the medical intensive care unit: An observational study" JOURNAL OF BIOMEDICAL INFORMATICS, ACADEMIC PRESS, NEW YORK, NY, US, vol. 38, no. 3, June 2005 (2005-06), pages 239-243, XP004892997 ISSN: 1532-0464
- WACHTER SB ET AL: "The employment of an iterative design process to develop a pulmonaa graphical display" JOURNAL OF THE AMERICAN MEDICAL INFORMATICS ASSOCIATION, vol. 10, no. 4, July 2003 (2003-07), - August 2003 (2003-08) XP008077457 cited in the application

## Description

### Technical field of the invention.

The method relates to a method and to a device for acquiring several changing values which are be monitored during the ventilation of the patient, and representing the values on a screen.

The mechanical ventilation of patients, as the case may be, is necessary to keep the patient alive.
This is for example the case with an anesthetic, or also with diseases or after an accident. A mechanical ventilation may however also damage the lungs of the patient. It is therefore the object of any ventilation treatment, to carry it out as briefly as possible and with the lowest possible intensity. Patient parameters and apparatus settings are to be used for estimating the necessity of ventilation, as well as the necessary intensity of the ventilation.

In order to adapt the ventilation where possible, to the requirements of the patient, a continuous, new assessment of the patient status and a likewise continuous, new assessment of the dependency of the patient on a mechanical ventilation is necessary. The dependency of the patient may be derived on the one hand from the present ventilation state, which may be recognized from the apparatus parameters set at the ventilator, and on the other hand from the patient status which is recognizable from the patient parameters.

### State of the art

Conventional ventilators, anaesthetic apparatus or monitoring apparatus have a screen as a display, on which patient parameters measured by way of sensors, and/or the settings of a mechanical ventilation may be represented in real-time.

The representation of the patient parameters may mostly be selected between a representation in the form of numeric values or in the form of graphs. Some of such graphs in each case show an individual patient parameter in course of time, wherein the x-axis represents the time, and the measured or computed value of the parameter at the given point in time is plotted on the y-axis. The curve therefore indicates a reading of a parameter at a point in time of the past which is displayed in each case, and in particular in real-time. Several such graphs may be represented next to one another or over one another. Two curves may be represented simultaneously in a single graph. Some graphs show two patient parameters which are plotted against one another in so-called loops. The values of the one parameter are plotted on the x-axis, and the values of the other parameter on the y-axis. In the course of time, a point representing in each case both readings runs along a closed curve, a loop so to say. These loops are continuously updated by way of this. The point representing both readings in each case shows the current values of the measurements in real-time.

The known graphic representations of such readings are therefore two-dimensional curves which represent one patient parameter, or two patient parameters in the special shape of loops in real-time. A changing of the reading effects a shape change of the loop or the graph.

The representation of readings of the patient parameters and/or of values computed therefrom are used by the physician for estimating the patient status. The physician estimates how the patient is doing at that moment, by way of comparing these readings and graphs to normal values and tolerable deviations from these, which are known by him.

The apparatus setting with a mechanical ventilation may be read off from a series of numerically displayed setting values. The assessment of these values is necessary, in order to be able to estimate the dependency of the patient on the mechanic ventilation and to achieve a rapid withdrawal.

Such numeric and graphic representations may only be assessed spontaneously with great experience. The physicians must deal with the displayed values in an intensive manner, in order to be able to correctly assess the situation. Overlooking an individual parameter which does not support a withdrawal, which encourages a reduction of the intensity or which supports an increase in the intensity, may lead grave problems.

Research work is published in the "Journal of the American Medical Informatics Association" Volume 10, Number 4 of July/August 2003 with the title "The Employment of an Iterative Design Process to Develop a Pulmonaa Graphical Display". In this, the development of a graphical display of the lung function for anaesthetics is specified as an example of an interactive fashioning method with an integrated usability test

With the design specified as an example, the usability test resulted in an increase of the intuitive perception ability of the anatomical significance of the representation by 25%. The intuitive perception ability of the significance of the variable representation means was increased by 34%, and the precision of the diagnostic content was only reduced by 4%. The design creating these best test values finally displayed a graphic element which comprises the following picture elements: a graphically, very simplified lung with two lung lobes, a graphically very simplified trachea with two bronchial branches which reach into the lung lobes, and an inflating bellows is represented connected to the bronchi, whose height represents the tidal volume. A rectangle is represented left of the bellows, whose colour represents the oxygen content of the respiratory air, and a rectangle is represented on the right of the bellows, whose height represents the end-tidal CO₂ values. The respiration rate is above the bellows as a number.

Triangles may be imaged in the tracheae and in the bronchi which symbolise a respiratory resistance. The colour of the lung lobe symbolises the oxygen supply of the lungs. The compliance of the lungs is represented by two different outlines of the lung lobes: an outline representing a foam bubble represents an increased compliance, a gridded cage represents a reduced compliance.

The graphic representation is not animated. It is specified as an important aspect in the description, that the intuitive perception ability of the representation may change when the representation is animated with real-time data. Future studies however have yet to show whether the representation remains intuitively perceptible and usable when it is animated with the patient data.

### Object of the invention.

It is therefore the object of the invention to simplify a diagnostic assessment of a patient undergoing ventilation, or to simplify an assessment of the parameters which are to be assessed with the ventilation of a patient. It is a further object of the invention to process and represent the parameters to be assessed. A further object of the invention lies in rendering the status of the patient or the dependency of the patient on the mechanical ventilation perceptible, in a manner which rapid and as intuitive as possible.

### Inventive solution of the object

According to the invention, this object is achieved by a method according to claim 1.

Such a method for detecting several, changing values of a different origin of a mechanically ventilated patient, and representation of the values on a monitoring screen, with the state of the art, has the feature that at least three of these values are acquired and qualitatively represented together in a single graphic element which encompasses a pictorial representation of the lung shape. It differs from this state of the art in that the current volume change of the ventilated lung is represented by way of the lung shape increasing and decreasing in size with each breath.

The animation of the lungs has the advantage, that the moving lung is directly understood as graphics representing the lungs of the patient. It indicates the rate, and may also qualitatively indicate the breath volume by way of the size change. Furthermore, one may directly recognise whether the lung does indeed move, i.e. whether the patient is indeed respirated, or whether for example an occlusion or a disconnection is present, in which case the lungs specifically advantageously have a fixed size.

The animation of the lung has the advantage compared to the animation of a bellows, that it is not the work of the ventilator which is rendered visible, but the effect of the ventilation.

If the lung shape is only able to experience size changes in one dimension, it is preferred for the lung shape in each case to be increased or decreased in size normally to a contour of the lung shape. This may be perceived in a very intuitive manner as a movement representing the respiratory movement. Such a size movement is perceived as an insufflation of the lungs and as an emptying of the lungs. It thus indicates a passive patient. The patient's own activity may be rendered visible in that the outline of the lung shape on the diaphragm side is changed in its fashion during the size change of the lung shape. The size change of the lung shape may therefore be effected in two ways, so that one may differentiate as to whether the patient actively breathes or is only ventilated. A trigger signal may be acquired in a manner known per se, for detecting whether the patient breaths actively.

The changing values are usefully measured on a patient being ventilated or possibly to be ventilated, by way of sensors. They may also be determined by calculation from such readings

Usefully the respiratory flow and/or the pressure conditions on the patient side are detected in a respiratory tube. With this, apart from the patient's own activity, one may also monitor as to whether a disconnection or occlusion is present. A constant value is allocated to the size of the lung shape, in the case that a disconnection or an occlusion is ascertained. The lung shape, in order to be able to intuitively differentiate the occlusion from the disconnection, is represented large in the case that an occlusion is ascertained, and in a small manner in the case that a disconnection is ascertained. One may envisage a normal lung shape size as a comparison value being superimposed on the moving or non-moving lung shape. This may e.g. be represented as a line or background area with the lung shape, with a size which is constant or a lung shape which increases and decreases in size. Such a line or area may likewise represent a comparison shape for the compliance of the lungs.

An airway shape is represented integrated into the lung shape, in the known manner. At least one changing value of a property of this airway shape, and which characterises the airway, is assigned to this airway shape. Preferably, the respiratory resistance is allocated to the width of the airway shape. Narrow airways are represented with a high respiratory resistance, and wider airways are represented with a low, i.e. normal breathing resistance. Apart from the width of the airway shape, the colour or the brightness level of the airway shape may also provide qualitative information on the respiratory resistance. Both properties may be combined with one another or used individually.

Furthermore, the graphic element may have a heart shape or a blood vessel shape, as also a lung muscle system in the form of a diaphragm below the diaphragm side of the lung shape. At least one changing value characterising the blood circulation is allocated to a characteristic of the heart shape or the blood vessel shape. Such values may be: blood pressure, represented by way of vessel width; oxygen saturation and CO₂ partial pressure, represented by two colours in part regions of the vessel- or heart shape; pulse, represented by a flashing or moving sign in the blood vessel shape or in the heart. A changing value characterising the participation of the patient in the respiratory work is allocated to a characteristic of this muscle system shape. Such a value may be a trigger signal, represented for example by way of a flashing-on of the muscle arch. The intensity of the participation may also be represented by way of the size of the muscle shape. Qualitative information on the activity of the lung muscle system may be provided in such a manner, which may be perceived in a rapid and intuitive manner. An activity of the lung muscle system is represented in several, for example in three, four, five or six graduations.

The change of the muscle system shape is effected usefully synchronously with the activity of the lung muscle system (strong-weak) and with the respiratory movement (inhalation, the arch becoming shallow; exhalation, arching of the muscle arch).

It is further desirable for the lung shape to provide qualitative information on the compliance.
Advantageously, this is accomplished by a contour line which is thicker and/or unequally thick with a stiffer lung, and which is thinner with a more compliable lung. The contour fashion of the lung shape may also provide qualitative information on the stiffness or expansibility (compliance) of the lung. With a stiff lung, this is represented e.g. in an angular manner, with a compliant lung, in a bloated or wrinkly manner. A patterning of the lung area may likewise represent the compliance. The patterning advantageously has an asymmetry if the value for the compliance does not lie in the normal range. The patterning may for example represent a grid, whose grid fields are more angular with a hard compliance, and round with a soft compliance. The characteristics of outline shape, patterning and contour line may advantageously also together qualitatively represent the compliance and draw attention to this. The lung should furthermore also be represented in a more asymmetrical manner with an increasing deviation from normal values, in order to render the deviation more obvious.

The pulse may furthermore by displayed by the rhythm of a contour change of the heart- or vessel shape, or of a brightness change or a colour change of the heart- or vessel shape.

Lungs with airways and muscle system shape together usefully form a single graphic element which is to be read as a whole. The individual constituents have a fixed relation to one another. Usually the muscles and the lung lobes move in a synchronous manner. If the patent breathes against the rhythm of the ventilator it can be observed that the movements of the muscle shape and the lung shape are asynchronous according to the asynchrony between lung an muscle. The vessel/heart shape is incorporated into this graphic element. This increases the intuitive readability of the symbols, since these are perceived as human organs. The readability of the lung shape as a lung increases the readability of the muscle shape as a diaphragm. The readability of these picture components again makes it clear to the observer, that the vessel shape represents a vessel, and the heart shape a heart. The close relationship of one shape to the other within the graphic element has further the advantage, that asynchrony of the patients activity and the mechanical ventilation becomes visible.

Apart from the patient parameters which are rendered easily perceivable by way of this summary in the organ representations, the physician as a rule also needs the apparatus parameters, in order to be able to correctly estimate the situation. These are better shown in second graphics. For this reason, it is suggested to represent a second graphic element on the screen, which graphically represents three, preferably more than three, particularly preferably all of the following parameters:
- the applied oxygen concentration "FiO₂",
- a positive endexpiratory pressure "PEEP",
- the inspiration pressure "Pᵢₙₛₚ" or in inspiratory mean pressure "Pᵢₘₑₐₙ",
- the ratio between controlled ventilation and spontaneous breathing "RRₜₒₜ/RRₛₚₒₙₜ, or the rapid, shallow breathing "RSB",
- the ratio of the theoretic minute volume (e.g. derived from IBW) to the current minute volume "MV"
- the variability index and/or
- an index for the breathing exertion of the patient (e.g. P0.1, which determines the breathing exertion on account of the vacuum generated by the patient, or an alternative index which determines the breathing exertion for example by way of the flow dynamics generated by the patient).

The physician, on account of these parameters or a selection-of-these parameters, may estimate how greatly the patient is dependent on the apparatus. These parameters too, should be able to be rendered perceivable as quickly as possible

For this purpose, these values are acquired and displayed in an analog or quasi-analog manner with a position of a division or of a positionally changing component on scales. Each scale is assigned to one of these parameters. The current value of each represented parameter is advantageously in each case shown on a range, said ranges in each case displaying poor values of the respective parameter at one end, and good values at a distance thereto, and these ranges are arranged in a manner such that the good values of all ranges are set in a relation to one another. This considerably simplifies the assessment of the displayed values.

The scale is advantageously divided into at least two regions. At least one region for good values and one for poor values, and as the case may be for middle values. These may differ by way of their colour, brightness or patterning. By way of the colour, brightness or patterning of one of these regions, one displays whether the displayed parameter value lies within or outside the region with good values. Advantageously, by way of the colour, brightness or patterning of a region of each range or of the background, it is displayed as to whether all displayed readings lie within the region with good values. If all values lie in the regions for good values, then one may consider disconnecting the patient from the ventilator. If at least one value is still not within this good region, then one may disregard to disconnect the patient from the ventilator.

Border line values of the regions may be fixed within the apparatus and therefore be unchangeable. In a preferred embodiment of the method, they may be pre-set, but they are defmable and/or adjustable by the user. To define or adjust the regions, the user defines or adjusts the border line values between one region and an adjacent region using an interface.

A device according to the invention and corresponding to the method described above is designed as follows:
The device, in a known manner, is provided with a screen, in order on this, to represent detected changing values with the ventilation of a patient, and comprises means for detecting at least three changing values of different origin, and means for representing the values. These means permit the acquired values to be qualitatively represented on the screen together in a single graphic element. This graphic element in the known manner, has a pictorial representation of a lung shape.

This device-however differs from the state of the art initially mentioned, in that the means for representing the values are designed such that a volume change of the ventilated lung which is detected with each breath, is represented in an animated manner by way of the size change of the lung shape corresponding to this corresponding volume change. Accordingly, it is not only a breath volume which is pictorially represented, but the effect of the ventilation on the lung is pictorially represented in real-time. For this, sensors are usefully provided. In a preferred embodiment, a sensor for monitoring pressure and/or the flow of the respiration air is present on the patient side on a respiration tubing. With this, one may ascertain whether a disconnection or an occlusion is present, or whether the patient is effectively ventilated. An animated lung is represented on the monitor when the lungs of the patient expand with ventilation air and this air compressed into the lung is let out again. In the two mentioned cases, in which the ventilation does not take place, the lung shape is however shown on the screen with a constant size. This renders visible the effect on the patient, and not the task set in the ventilator.

Usefully, the constant size of the lung shape is large in the case that an occlusion is ascertained, and small in the case that a disconnection is ascertained. The surface change of the lung shape representing the breathing is advantageously in a relation with the measured tidal volume.

The represented lung shape advantageously also has a airway shape (trachea with bronchi). At least a changing value relating to the airways, in particular the resistance, is represented with this. A current ventilation resistance may also represented, or the deviation from a normal value may be emphasised, by the colour or the brightness level of the airway shape.

The lung shape may be divided up. An area division of the area of the lung shape advantageously represents a difference between an ideal lung volume and an actually ventilated lung volume. Such a division - spoken pictorially - is perceived as "water" in the lung and advantageously contains information as to which part of the lung does not participate in the breathing.

A current design of a contour line of the lung shape and/or a current configuration of an outline of the lung shape advantageously contain qualitative information on the stiffness or the compliance of the lung. This contour line and this outline are animated. They become larger and smaller with each breath. As already described, the counter line has a thickness corresponding to the compliance, and the outline configuration is more straight-lined and angular, the stiffer is the lung, and is rounder and more indented, the more compliant is the lung. A comparison value in the form of a taut, round line or such a background surface may be blended in, for comparison purposes. This represents an ideal compliance as a line following the outline of the ideal lung shape, or an area having the ideal lung shape. With this, one may furthermore represent an ideal breath volume.

Part of the graphic element is furthermore advantageously a muscle system shape. With this muscle shape usefully represented as a diaphragm represented below the lung, one may represent a changing value concerning the participation of the patient in the breathing work. Such a representation may be read in an intuitive manner. A thick muscle for example may be recognised as a large participation, and a thin one as a weak participation. The appearance of the muscle shape and the absence on the muscle shape are directly recognisable as a trigger signal of the patient, or as an absence of this.

A further constituent of the graphic element is advantageously a vessel shape or heart shape. This is suitable in order to represent a changing value concerning the blood circulation. A current blood pressure or any other haemodynamic index, in particular the current average blood pressure, may be represented by the current width of the vessel shape. A current oxygen saturation or any other index of oxygenation, and the current CO₂ partial pressure of the blood or any other index of ventilation may be represented by way of the current colour, at least of one region of the vessel shape and/or the heart shape.

The pulse may advantageously be indicated by way of a symbol which runs along a vessel shape or which flashes.

It may generally be stated that at least three different first graphic parameters corresponding to the origin, may be allocated to at least three values of a different origin, wherein first graphic parameters define different graphic basic characteristics of the element and of the background. The following are to be understood for example as graphic basic characteristics of the represented element and its background:
■ a position of the element on the screen or in relation to another element
■ a basic shape
■ a contour line
■ a colouring
■ a brightness
■ a patterning
■ a division or
■ a positionally changing constituent, etc..

These basic characteristics may in each case relate to the characteristic of the element and/or of the background.

The advantage of this association of the origin with the basic characteristic of the element or its background is that 3, 4, 5, 6 or more data each of a different origin are represented and are furthermore capable of being qualitatively assessed, in a single element. This is because an element has the above different basic characteristics, to which in each-case a particular origin of the data may be allocated.

The qualitative representation of the reading or the apparatus setting is then effected on account of the value of the represented parameter. Second graphic parameters which define different conditions of the respective basic characteristic are specifically allocated to different values of the same origin. As conditions of the basic characteristics, one may mention:
■ size and design of the shape,
■ thickness of the contour line,
■ defined colour from a colour scale,
■ specific brightness from a graduation of the brightness, in each case of the element area, of the background, of a contour line, of a moving component, of a part area, etc.,
■ design of a patterning on an existing area,
■ contrast of the patterning,
■ positioning of the present division, definition of the division, width of the border region,
■ position of a present positionally changing component, etc..

If an assessment of one or more of the represented values with respect to a comparison value or a comparison value range is displayed, then this has the advantage that the assessment of the value may be effected in a quicker manner, and thus measures may be initiated more quickly, which is in the interest of the patient.

### Brief description of the figures

Examples of a representation achieved with the method are shown in the figures. The shapes and associations according to the description and the figures are exemplary and may be changed without departing from the invention defined in the claims. There are shown in:
- Fig. 1: a screen of a ventilator with a representation of 14 values generated with the method according to the invention, and additional valuations of these values in the form of a schematic organ representation for the patient parameters, and a comparison scale bar for apparatus parameters which indicate the dependence of the patient on the ventilation.
- Fig. 2: the same screen after opening an alarm window.
- Fig. 3: a screen of a ventilator with the representation generated with the method according to the invention, and with an additional, conventional representation of two patient parameters.
- Fig. 4: a screen section which represents the comparison scale bar with readings displayed therein, wherein several readings lie in a range which requires a continuation of the machine support of breathing.
- Fig. 5: the screen section according to Figure 4, wherein all readings lie in a range which could permit the termination of the machine support for breathing.
- Fig. 6: a screen section which represents the organ representation with the readings displayed therein, wherein normal lung volume, a normal functional residual capacity, a poor compliance, a relatively high breathing resistance, a large muscle force and a high blood pressure are represented.
- Fig. 7: the screen section according to Fig. 6, wherein however an unhealthy functional residual capacity, a normal compliance, a poor breathing resistance, a low muscle force and a low blood pressure are represented.
- Fig. 8: the representation of a normal volume by way of the element size, with a normal compliance, represented by the element shape and the fme contour line.
- Fig. 9: the representation of a low lung volume by way of the element size, in comparison to a comparison line or comparison area, with normal compliance.
- Fig. 10: the active breathing of the patient by way of the lung shape change, in comparison to a comparison line or comparison area.
- Fig. 11: the represented picture with absent readings (only comparison line or comparison area).
- Fig. 12: a normal, functional residual capacity by way of division of the element area.
- Fig. 13: an unhealthy, functional residual capacity by way of division of the element area.
- Fig. 14: the represented picture with absent readings.
- Fig. 15: a poor compliance (hard lung) by way of an asymmetrical, angular shape, and a contour line of the element whose thickness is variable.
- Fig. 16: a normal compliance by way of a symmetrical outline shape with a taut, round leading of the lines.
- Fig. 17: an excessive value for the compliance, represented by the asymmetrical, shrivelled bloated shape which partly reaches beyond the comparison area, and the very thin, almost absent contour line of the element.
- Fig. 18: a normal breathing resistance by way of a complete filling of the element with a colour, and brightness of the colour.
- Fig. 19: an increased breathing resistance by way of the distance between the coloured filling and the contour line of the element, and a brighter colour.
- Fig. 20: an extremely high breathing resistance by way of a thin coloured area and its still brighter colour.
- Fig. 21: the element with the absence of the reading for the breathing resistance.
- Fig. 22: a low muscle activity of the patient by way of a lower thickness of the element.
- Fig. 23: a high muscle activity of the patient by way of a large thickness of the element.
- Fig. 24: a trigger signal without measurable muscle activity.
- Fig. 25: a low blood pressure by way of the reduction of the diameter of the represented blood vessel, from the left to the right.
- Fig. 26: a high blood pressure by way of the enlargement of the diameter of the represented blood vessel, from the left to the right.
- Fig. 27: a normal blood pressure.
- Fig. 28: the absence of a reading for the blood pressure.
- Fig. 29: the pulse by way of a moved element on the standing picture for the blood vessel.
- Fig. 30: the saturation of the blood with oxygen by way of the colour of the blood on the right side of the blood vessel.

### Detailed description of the invention by way of the embodiment examples represented in the figures

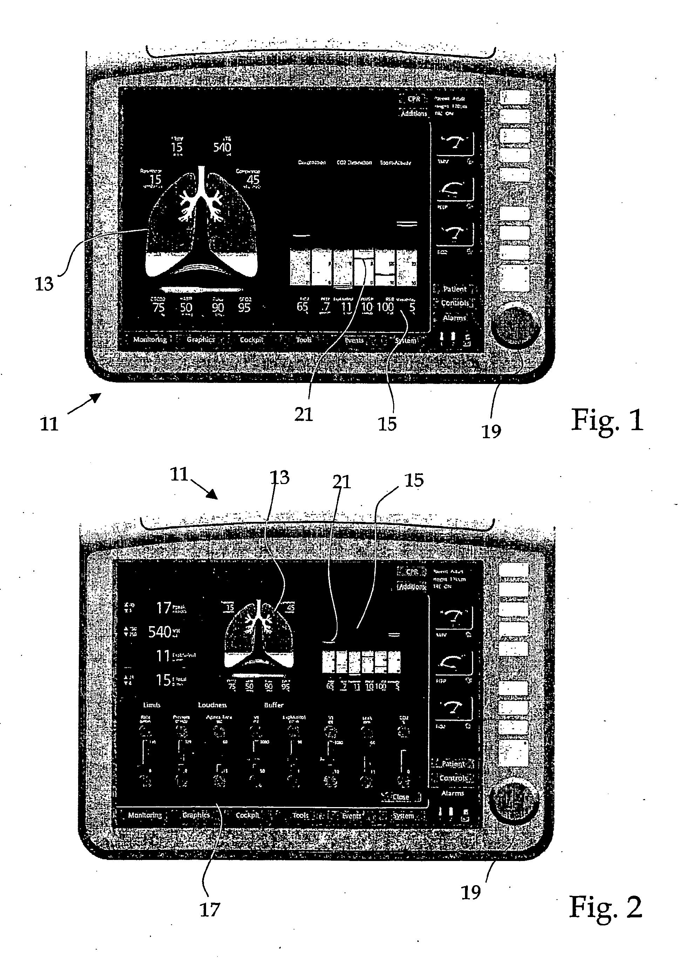

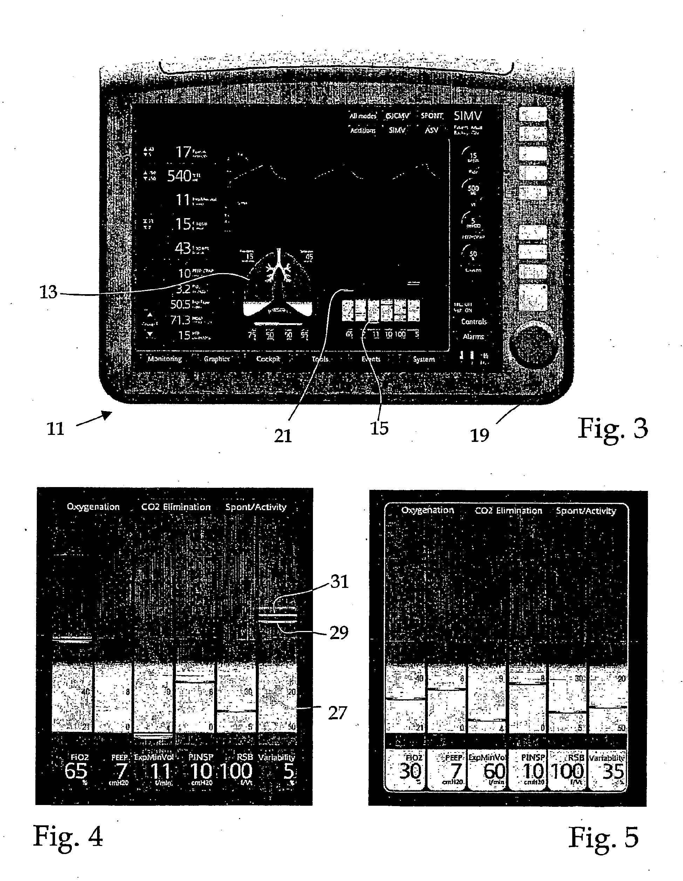

The screens 11 represented in the Figures 1 to 3 show representations generated with the method according to the invention. These representations comprise a first region 13 with patient parameters which contains an element reminiscent of an organ, and a region 15 with apparatus parameters which has a scale bar. The scales of this scale bar are different. Readings and apparatus settings are displayed on these scales with a positionally changing component 21. In Figure 1, these two regions 13 and 15 practically take up the complete screen. In Figure 3 they are squeezed together in the upper half of the screen, since a window is opened around the lower region. Such a window may be selected and opened with the selection button 19, whereupon the representation according to Figure 1 changes into a representation according to Figure 2. In Figure 3, the representation of both regions mentioned above only takes up a part region of the screen, whilst an upper part region presents two graphs in a conventional manner, and a left screen region is filled with numeric values.

These different screen designs may be provided specific to the apparatus, or may be selectable with an apparatus.

The region 15 in which the apparatus parameters processed for an easier interpretation are represented, are shown in Figures 4 and 5. In the example, the following apparatus parameters are specified (from left to right):
FiO₂, the oxygen content of the respiration air, in percent;
PEEP, the positive end-expiratory pressure in cmH₂O;
ExpMinVol, the expiratory minute volume in litres per minute;
P_{INSP}, the inspiratory overpressure in cmH₂O;
RSB (rapid shallow breathing), an index for the shallowness or efficiency of the breathing in f/Vt (breathing frequency divided by tidal volume);
variability, an index which indicates the deviation from an absolutely regular breathing;
an index which indicates the breathing exertion. A known such index is called P0.1. This is measured on account of a vacuum in the ventilation system which is achieved over a short time period by way of the activity of the patient. An index comparable to P0.1 may be obtained by way of measuring dynamics of the flow of the ventilation gas created by the patient.

The first two scales provide information on the oxygen supply to the patient, which is why they are titled "oxygenation", the middle two for ventilation, which is why they are titled "CO₂-elimination", and the last two for the patient's own initiative, which is why they are titled "Spont/Activity".

Each scale is directed in a vertical manner, and comprises a normal range 27 in which the values maybe estimated as being favourable for the patient or for the course of the illness. This range is coloured and is highlighted with regard to brightness. In the example it is bright green, whilst the rest of the scale is dark grey and only highlighted very weakly from the black background of the screen. The exact reading is specified numerically below each scale. The same value is displayed in an analog manner with the help of a positionally changing indicator 29 within the scale.

In Figure 4, the readings which are displayed on the scales for PEEP, P_{INSP} and RSB each lie within the normal range 27. For this reason, these normal ranges illuminate in a brighter green that the adjacent normal ranges of the scales, which indicate a range which is not to be classified as healthy or normal. In Figure 5, all readings are within the normal ranges. For these reasons, all normal ranges illuminate in this brighter green. Furthermore, the background of the digital displays likewise illuminates in this green, in order to indicate that all scales indicate values which lie within the normal range 27.

Preferably, a single parameter with a reading within the normal range is not highlighted by colour, which is different to the shown variant. In contrast, the time duration since the occurrence of the reading in the normal range is displayed. In the case that all parameters have readings in the normal range, then all normal ranges together are highlighted by colour. A first blue indicates that not all readings yet lie within the normal range. A blue which is lighter compared to this, indicates that all readings lie within the normal range, and a withdrawal may be considered.

This apparatus parameter region 15 thus comprises six scales arranged next to one another, of which in each case two scales lying next to one another are grouped together, at least by a title or a common frame. The six scales, as shown in Figure 5, are grouped together with a common frame. One scale, together with the numeric value detail, may be considered as an element. The following values are represented in this element: the origin of the readings accordingly defines the position of the scales with respect to the other scales. Each scale is an element with a background of a numerical display (changes from black to green) with a number thereon (changes from white to black), with a normal region highlighted by colour (changes in its brightness), with a positionally changeable indicator 29 (changes its position on the scale), with a trail 31 for the display of a tendency (depending on the tendency of the position change, is above or below the indicator 31, and may be shorter or longer depending on the speed of the value change (or position change).

The reading or setting value for the scale provided at a certain location is therefore represented in an analog and numerical manner (the agreement of this two display types is unfortunately not always given in the figures). The analog representation is effected via the position of the positionally changing indicator 29.

A value with a second origin is the fact as to whether this reading lies within or outside the normal region. This fact may also be represented by the brightness of the normal range 27, which has been highlighted by way of colour.

A value with a third origin is the fact as to whether all remaining values likewise lie within the respective normal range 27. This fact may be represented by way of the brightness and the colour of the background of the numbers of the numeric display, and the number in front of this background, and/or by way of the brightness or colour of all normal ranges.

A value with a fourth origin is a tendency of the development of the displayed value. This tendency may be displayed by a trail 31 of the positionally changeable component 29.

A value with the fifth origin is the speed of the change in this direction, said speed being able to be displayed by the length of the trail.

Therefore, with a glance of a single element, one may recognise which value e.g. for FiO₂ is set, whether this value lies in the normal range, in which direction the last setting correction was made, and, if FiO₂ lies within the normal range, whether the other readings- and setting values already likewise each lie in the normal region.

In the case of ExpMinVol, there are scale regions on both sides of the normal range 27, which display unfavourable values. These normal ranges 27 are at the end with the remaining scales. In contrast to the design represented in the figures, with individual or all scales, one may also add a transition region, differentiated by another colour, patterning or another brightness, between the normal range and the unfavourable ranges of the scales. The normal range 27 is merely blurred with respect to the unfavourable range in the represented embodiment.

The scales are compressed or extended in a manner such that the normal ranges have a unitary length. The scales on the other hand assume differently long ranges of the element length. A clear representation results by way of this, with which the approximation of an individual value to the normal range may be recognised in an excellent manner. Such an easy readability of the display may not be achieved with differently long normal ranges 27.

The normal ranges of the scales are arranged on a central axis, common to all. This would also increase the clarity of the display if the normal regions were to have different lengths

The readings which are represented in the scales of the elements and displayed therebelow in a numeric manner, are partly readings and partly setting values of the ventilator. The oxygen content of the ventilation gas FiO₂ may be reduced before the setting of the apparatus, or by way of a measurement by way of a sensor.

PEEP is usually likewise a setting value. ExpMinVol, the exhaled volume per minute, is computed from the flow measurement during the expiration phase, and averaged over several breaths.

Pinsp is mostly a setting value. This pressure may however also be monitored with a sensor. RSB, "rapid shallow breathing" is computed from measurements. It is based on a measurement of the rate and of the breathed volume.

The variability is computed on account of the differences of the temporal distances of the breaths, the differences in the length of the breaths and/or the volume differences with the breaths. A good variability indicates a larger independence of the patient from the ventilation, less variability however an larger dependency.

The region 13 with the representation of the patient parameters has a pictorial and qualitative display, which is accompanied by a numeric and quantitative display of a few readings. The numerical specification of the readings may also be made at a different location. It is evidently not a constituent of the graphic elements, which permits an easy readability and interpretation ability of the readings.

The representation of the patient parameters in the region 13 includes a schematic lung 31 with two lung lobes. The lung lobes are formed in a symmetrical manner with a healthy compliance. They both contain the same information. Furthermore, the representation includes a schematic airway tree 33, a schematic muscle system 35 and a schematic blood vessel 37. These part regions contain different amounts of information. They are described in detail further below.

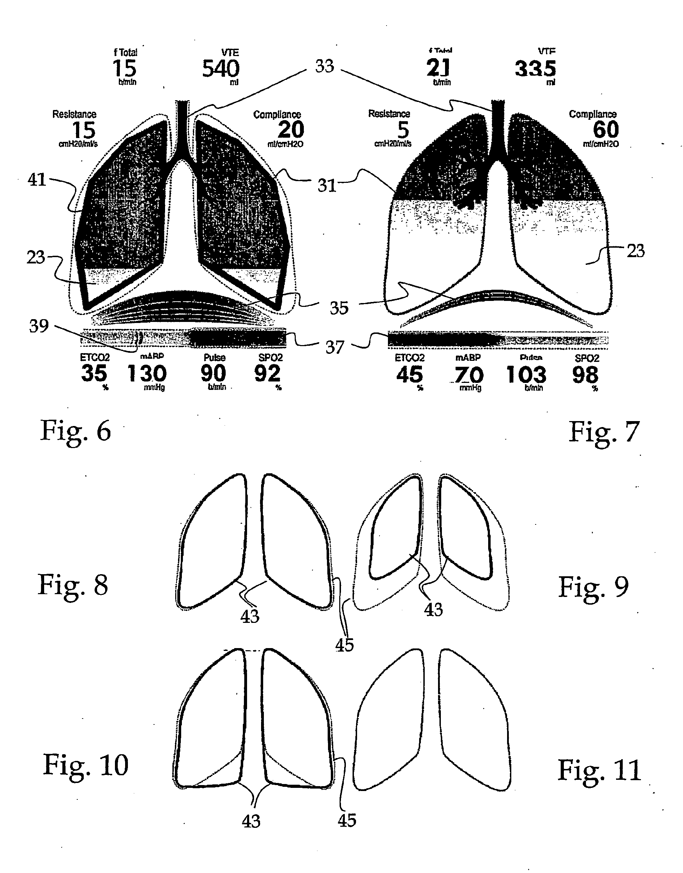

Two exemplary designs of the graphic element represented in the regions 13 are compared in Figures 6 and 7. Differences of the shaped formation of the element are immediately evident to the eye, whereas the number values around the graphic elements must firstly be read and then need to be interpreted.

In comparison to the conventional processing of readings and the setting values by way of graphs and numerical values, the advantage of the processing according to the invention becomes quite apparent by way of this representation of the respective data. Whilst the association of the number values and readings, as well as the graph forms is only possible via learned positional association, shape estimations or reading the lettering, such an association may be intuitively recognised with the representation of pictures which are reminiscent of organs. A difference 23 from an ideal total capacity of the lung or the muscle activity of the muscle 35 may for example be recognised immediately in the pictorial representation, whereas a number value would be significantly more difficult to interpret. The compliance for example is represented as a number value and as a graphic design of the element The angular and thick contour line 41 in Figure 6 evidently shows the hardness, whereas the round shapes being taut, and the fineness of the contour line in Figure 7 may be recognised immediately by anyone as the healthy compliance of the lung. The association of the values 20 ml/cmH₂O or 60 ml/cmH₂O with a corresponding state of matters is however much more abstract even for experienced physicians, and thus may be recognised less quickly and reliably. It requires thought work, which thanks to the new type of processing of the data, may be invested to greater use in the consideration and the selection of therapeutic measures.

### The lung 31:

The lung shape 31 consists of two mirror-imaged lung lobes. The lobes with the shown example are formed identically in a mirrored manner. They may however also be formed in an unequal manner, in order for example to make room for a heart between them.

The size of the lung lobes very coarsely indicates the lung volume. In Figures 8 and 10, the lung is filled with air, in Figures 9, it is in the exhaled condition.

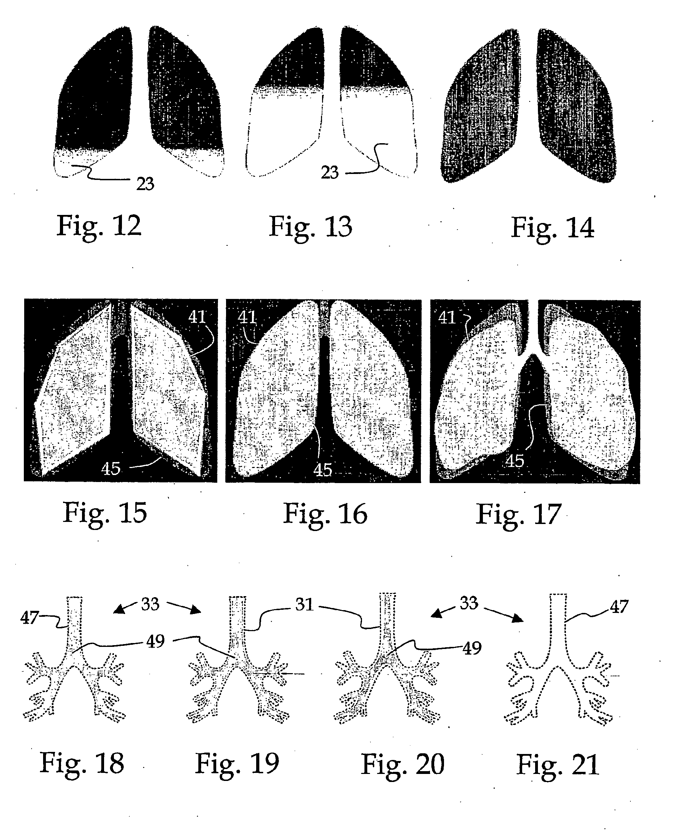

The lung lobes have an outline form. This outline form is designed differently, depending on the compliance of the lung. The shape of the lung is therefore allocated to the reading origin "compliance". A poor compliance is shown in Figure 15, a normal one in Figure 16, and a compliance which is too large in Figure 17. The compliance additionally to the representation via the outline shape, is also represented via the contour line 41. A low compliance may be recognised by a thick contour line which is straight-lined and angular. A high compliance, i.e. a flexible lung may be recognised by the thin contour line along a shape which is inflated with respect to the normal shape. Five or six graduations are provided: one for the flexible lung, one for the normal lung and three or four for differently stiff lungs. The compliance of the lung has a large band width and may be determined afresh with each breath.

With active breathing, the lung shape is different depending on the breathing condition of the lung. The lung is inhaled in an active manner in Figure 10. The inner corners 43 of the lung lobes are pulled downwards, so that the lung lobes end at the bottom on a common straight line. On exhaling, the lung area decreases and the inner corners of the lung lobes return to the exhaled condition according to Figure 9. In this condition, the inner corners 43 of the lung lobes are receded to the top, so that the lower edges of the lung lobes rise towards the middle.

The lung shape remains similar with an inactive patient. It then merely changes its area, in that the lung shape is spread from a centre (e.g. at the upper lung lobe tip).

E.g. 10 levels from Figure 9 to Figures 10 or from Figure 9 to Figure 8 are provided for the representation. The area of the lung increases synchronously with the volume curve on inhaling and reduces on exhaling, according to the current flow measurement

In Figure 11, the lung shape is represented as is indicated with the absence of readings.

For this reason, the origin of the values of the activity/passivity of the patient is allocated to the breathing shape change of the lung. The size of the lung, or the breathing size change indicates the respiratory condition of the lung.

In each case, the contour 45 of a healthy, inhaled lung as a comparison value, is drawn in the Figures 8 to 11 and 15 to 17. In the representation of the readings, this comparison line serves for the assessment of the displayed lung size and the displayed compliance. The shapes and the size of the lung may be read off in an improved and unambiguous manner by way of this.

The lung lobes assume an area and have a coloured division of the area (Fig. 12 and 13). The representation according to Figure 14 indicates that no measurement of the functional residual capacity is present. If no measurement is available, the lung is grey and the area is undivided. If a measurement is available, the lung is divided into an upper blue, slightly patterned region, and into a lower, white region 23. The transition is shown in blurred manner. The division is designed in a different manner, depending on the size of the functional residual capacity (FRC) of the lung. The representation according to Figure 12 still indicates a normal FRC, the representation according to Figure 13, a poor FRC. The display is supported with new FRC readings after every approx. 60 minutes.

The colouring and the upper share of the lung are therefore allocated to a breathing volume measurement and to a measurement of the functional residual capacity, which are compared to an ideal lung volume (upper plus lower share) which is computed on account of the body size of the patient. The readings are mirrored in the position of the division defined by respective graphic parameters.

The parameters of tidal volume, functional residual capacity, compliance, lung size, active/passive breathing, and breathing condition of the lung, and thus also the breathing frequency may therefore be practically perceived at a single glance in the form of a temporal sequence of differently large shapes. The different parameters superimpose in the representation. The lung with a low compliance may likewise change its size and shape in the case that respective data are available, as with a lung of normal or high compliance. The movement of the lung lobes participate in the division of the lung into a difference 23 from an ideal total capacity and the given total capacity, and is independent of the compliance. The share of the functional residual capacity fills the lung lobes above the division, when these appear in expired representation (Fig. 9). In an inspired representation (Fig. 12 or 13) the upper share includes the present total volume of the lung. In a preferred embodiment, accordingly at least 4 or 5 different parameter are each represented in a qualitative manner.

### The airway tree 33:

A graphic element belonging to the lung is the airway tree 33. The airway tree 33 has a trachea which is represented in a simplified manner, with two bronchi and further branching. The basic shape of this airway tree already remains unchanged and is represented as a comparison value with the contour line 47. This airway tree graphically represents the breathing resistance, or resistance. The readings are represented in the form of a coloured airway tree 49 in the inside if this basic shape. With readings for a high respiratory resistance, the coloured airway tree 49 is designed in a thin-branched manner, whilst with a low respiratory resistance, the readings are represented as a thick-branched airway tree 49. Accordingly, the white line which fills the intermediate space between the inner, coloured airway tree 49 and the contour line 47, is thick with a high respiratory resistance, and is thin or absent with a low resistance. The brightness or colouring of the inner airway tree 49 is adapted to the respiratory resistance. With a high resistance, the colour is brighter and/or less intensive than with a low resistance. A healthy to pale pink is preferred as a colour. Such adaptations may however also be effected in a manner different to that described. Thus the airway tree may be represented in an alarm colour with a high resistance.

The measurement of the breathing resistance may be made with each breath. It generates data with a band width of 0 to 50 cmH₂O/ml/s. Three levels are provided for the representation of the resistance. No coloured airway tree 49 but only the contour of the basic shape is represented with the absence of readings.

### The diaphragm 35:

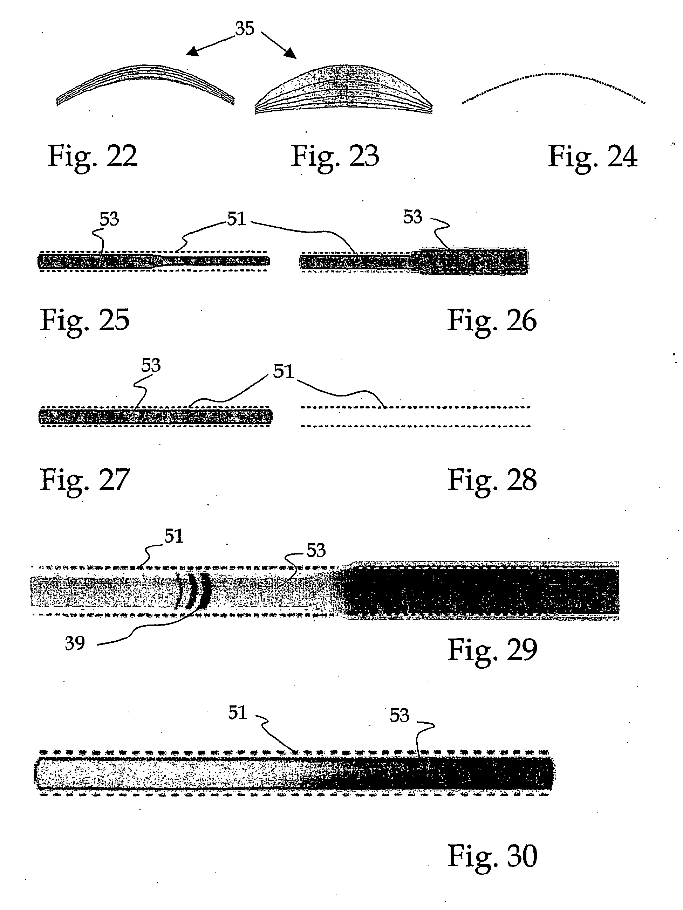

The muscle 35 represented as a stylised diaphragm may likewise be counted as belonging to the lung. The dimension of the muscle 35 indicates how greatly the patient breathes himself. A strong muscle according to Figure 23 shows a large participation of the patient in the breathing work. A weak muscle according to Figure 22 shows a poor participation of the patient in the breathing work. The absence of the muscle indicates that the patient does not participate in the breathing work, or that no readings are present. A muscle line according to Figure 24 indicates that a trigger signal is present. This muscle line illuminates with a trigger signal and fades again thereafter.

The muscle 35 changes its shape with patient activity, but remains equally strong. It extends on breathing in, and curves on breathing out. The shape of the lung lobes moves parallel thereto, as described. In each case, 10 levels between the inhaled and exhaled lung are provided for both muscle strengths for the representation of the movement.

### The blood vessel 37:

The representation of values with regard to the blood, specifically the mean blood pressure, the pulse and the saturation of the blood with oxygen, for example the arterial CO₂-partial-pressure is graphically independent of the lung, airways and muscle system. These values are represented by a stylised blood vessel 37. A heart may also be represented instead of a blood artery 37.

The blood vessel 27 has a left and a right side, which in the representations according to Fig. 25 to 30 are only separated by the brightness of the areas and/or by the shape. In reality, a coloured difference is also present, which however is not pictorially represented in this document. For the case that no readings and no pulse are present, only the contour line 51 of the blood vessel according to Fig. 28 is displayed. If no reading is present for the oxygen saturation of the blood, but only one for the blood pressure, the area is represented grey (Fig. 25-27), in order to be able to indicate the blood pressure. If only one pulse is displayed, the blood vessel is filled in grey, as is represented in Figure 27.

The gas exchange is effected schematically in the middle of the blood vessel. This middle may be marked with a shape, e.g. with a circle, a point or a lung shape. A positionally changeable component (Fig. 29) in the form of a pressure wave symbol runs with the rhythm of the pulse from left to right. The blood "runs" in the same direction. This means: the blood is represented before the gas exchange left of the middle. There, its colour, as the case may be, can indicate the arterial CO₂-partial-pressure. The blood is represented after the gas exchange to the right of the middle. There, the colour indicates the oxygen saturation of the arterial pressure. The reading which indicates the CO₂-partial-pressure, is qualitatively shown in two graduations of the colour blue. The reading which indicates the oxygen saturation, is qualitatively shown in two graduations of the colour red, specifically pink and magenta. A high oxygen saturation is represented by a full magenta of the right blood vessel side in the Figures 29 and 30.

A haemodynamic evaluation is represented by the width of the coloured or grey filling area 53. It is suggested to leave the width of the filling area 53 constant in the left part of the blood vessel. The width of the filling area 53 is varied on the right side of the blood vessel, in order to qualitatively display the average arterial blood pressure. A wide formation of the right-side filling area 53 beyond the width of the filling area 53 on the left side, and a comparison contour 51 of the blood vessel, indicates a high blood pressure reading (Fig. 26). A narrow, right filling area 53 however indicates a lower reading for the blood pressure (fig. 25). A normal blood pressure is displayed with a constantly wide filling area 53 (Fig. 27).

The blood pressure may also be indicated in a manner which differs from the above cited manner. It is e.g. possible, according to the blood pressure, to narrow or widen the shape of the blood vessel as a whole from the left to right, to inflate or contract a region. The blood pressure could be represented over the whole length of the blood vessel by the width of the filling area 53, a thickness of a vessel contour line, or another changing characteristic of the representation.

One differentiates between only four representations: high blood pressure, normal blood pressure, low blood pressure, no measurement. The representation is concluded in each case after 5 to 60 minutes on the basis of new readings. It represents the average arterial blood pressure and has a band width of 0 to 250 mmHg.

If no measurement of the blood pressure is present, the saturation of the blood or the respective arterial partial pressure may for example be represented by way of a coloured point on the left and right side (not shown in the figures).

For this reason, the following three parameters are represented in a qualitative manner with the help of the blood vessel 37, e.g. by way of the shape, the colour and a moving component: blood pressure; oxygen saturation, pulse. Furthermore, one may possibly represent the CO₂-partial-pressure.

The values for the compliance and the resistance are determined via a measurement of flow and pressure on breathing in and out, and displayed numerically as well as graphically. These measurements are for example made with a two-way flow sensor which is close to the patient, and the pressure sensors belonging to the ventilator, and converted in the known manner, in order to obtain values for the compliance and the resistance. The determined values, then according to their magnitude, are assigned to one of six (compliance) or one of three (resistance) different graphic parameter values.

Values for the number of breaths per minute are taken from the ventilator and, as the case may be, are combined with trigger signals activated by the patient. For example, a time measurement is taken over eight breaths, and converted into a number of breaths per minute. This computed value is rounded, and numerically displayed in whole numbers. The representation of the lung movement however shows the breathing movement synchronously with a volume which is determined with the flow measurement mentioned above. Individual short breaths as well as pauses in the breathing movement are represented as such.

The expiratory tidal volume VTE is determined by way of integration of a flow measurement over the expiration phase. This measurement and computation value forms the basis for a numerical display of a volume, but also for the graphic, pictorial representation of the lung size. One applies the body size for computing the pictorially represented lung size. The lung size is composed of the tidal volume and the functional residual capacity (upper share), as well as a difference to an ideal lung size. The tidal volume is measured with each breath. The functional residual capacity is determined by a manoeuvre, in which specifically the oxygen concentration in the respiration air is changed in a defined scope, and the effect on the CO₂-content and the O₂-content of the exhaled air is measured. Then, on the basis of these readings and setting values, one may draw conclusions on the functional residual capacity by way of calculation.

The upper share of the total area of the lung representation is then computed from the tidal volume and the functional residual capacity, and the respective area shares from the difference to an ideal lung volume. On breathing in and out, the volume of the respiration gas is measured and changed according to the size of the lung representation. With this adaptation of the lung size on breathing in and out, in each case one applies the most recent evaluation of the tidal volume and of the residual capacity, and the currently measured respiratory volume is added or subtracted. The maximum lung size is independent on the actual, ideal lung size in comparison to the IBW. The change of the lung size there represents a percentage change.

The CO₂-content of the expired breathing air is measured with a suitable sensor. The CO₂-partial-pressure of the blood may also be measured transcutaneously, as the case may be, also invasively. This reading is displayed in a numeric manner, but is also used for determining the functional residual capacity with the help of the described manoeuvre.

The blood pressure is measured sporadically with a sleeve, or invasively with a catheter. The average arterial blood pressure mABP is determined from the readings. This reading is numerically displayed in an exact manner, but only allocated to three graphic parameter values, specifically low, normal and high.

The oxygen saturation and the pulse are measured with a pulse oximeter. Invasive or transcutaneous measurements are however also possible. Instead of the saturation, one may also measure a partial pressure. The reading for the oxygen content of the blood is displayed in a numeric manner, but only allocated to two graphic parameters, specifically poor and good. The reading for the pulse is converted to a pulse rate per minute and displayed numerically. Each pulse stroke however causes the pressure wave symbol 39 to begin to run from the left to the right over the length of the blood vessel 37. The speed of the symbol 39 may be changeable, and increase and reduced according to the pulse rate.

## Claims

1. A device with a screen, in order on this to represent acquired, changing values with a mechanical ventilation of a patient,
with means for acquiring at least three changing values of different origin, and
means for representing the values, which permit the acquired values to be qualitatively represented together on the screen in a single graphic element,
said graphic element including a pictorial representation of a lung shape, a current design of the lung shape containing qualitative information on the compliance of the lung,
**characterised in that** the means for representing die values are designed such that a volume change of the ventilated lung which is acquired with each breath, is represented in an animated manner by way of a size change of the lung shape corresponding to this volume change, involving an animation of a contour line of the lung shape, the design of which containing qualitative information on the compliance of the lung.

2. A device according to claim 1, **characterised in that** a sensor for monitoring pressure and/or the flow of ventilation air is present, in order to be able to ascertain a disconnection or an occlusion, and that in both cases the lung shape is represented with a constant size on the screen, the constant size of the lung shape being large in the case that an occlusion is ascertained, and the constant size of the lung shape being small in the case that a disconnection is ascertained.

3. A device according to claim 1 or 2, **characterised in that** the area change of the lung shape representing the breathing is in relation to the measured tidal volume.

4. A device according to one of the claims I to 4, **characterised in that** an area division of the area of the lung shape is present, which represents a difference between an ideal lung volume and a ventilated lung volume.

5. A device according to one of the claims 1 to 4, **characterised in that** an ideal lung volume, and/or an ideal compliance is represented as a line following the contour line of the ideal lung shape, or an area with the ideal lung shape which is stepped with regard to colour or brightness.

6. A device according to one of the claims 1 to 5, **characterised in that** the represented lung shape also includes an airway shape, and a current ventilation resistance is represented by way of the current width of the airway shape and/or by the colour or brightness level of the airway shape.

7. A device according to one of the claims 1 to 6. **characterised in that** the graphic element also includes a muscle system shape which represents a muscle system, in particular a diaphragm, and at least one changing value concerning the participation of the patient in the breathing work is represented with the muscle shape.

8. A device according to one of the claims 1 to 7, **characterised in that** the graphic element also includes a vessel shape and/or heart shape, and at least one changing value concerning the blood circulation is represented with the vessel shape and/or heart shape.

9. A device according to claim 8, **characterised in that** a current blood pressure, in particular the current average blood pressure is represented by the current width of the vessel shape and/or the heart shape.

10. A device according to one of the claims 8 or 9, **characterised in that** a current oxygen saturation of the blood is represented by way of the current colour at least of one region of the vessel shape and/or the heart shape.

11. A device according to claim 8 to 10, **characterised in that** a symbol of the pulse which runs along a vessel shape and/or the heart shape or which flashes is displayed.

12. A device according to one of the preceding claims, **characterised in that** a second graphic element is represented on the screen, which graphically represents at least three, preferably more than three, particularly preferably all of the following parameters:
- the applied oxygen concentration "FiO₂",
- a positive endexpiratory pressure "PEEP",
- the inspiration pressure "Pᵢₙₛₚ" or in inspiratory mean pressure "Pᵢₘₑₐₙ",
- the ratio between controlled ventilation and spontaneous breathing "RRₜₒₜ/RRₛₚₒₙₜ", or the rapid shallow breathing "RSB",
- the ratio of the theoretic minute volume (e.g. derived from IBW) to the current minute volume "MV"
- the variability index and/or
- an index for the breathing exertion of the patient.

13. A device according to claim 12, **characterised in that** the parameter values are displayed in an analog or quasi-analog manner with a position of a division or of a positionally changing component, on scales allocated to the parameters.

14. A device according to claim 12 or 13, **characterised in that** the current value of each represented parameter is represented in each case on a range, said ranges in each case displaying poor values of the respective parameter at one end, and good values at a distance thereto, and these ranges are arranged in a manner such that the good values of all ranges are graphically put in a relation to one another.

15. A device according to one of the claims 12 to 14, **characterised in that** at least one region with good values, a region with poor values, and as the case may, a region with middle values are represented, and are different by way of their colour, their brightness and/or by way of their patterning, wherein by way of the colour, brightness or patterning of a region of each range and/or of the background, it is displayed as to whether all displayed readings lie within their region with good values.

## Patentansprüche

1. Vorrichtung mit einem Bildschirm, um auf diesem mit einer mechanischen Beatmung eines Patienten erzielte, wechselnde Werte darzustellen,
mit Mitteln zur Erzielung von wenigstens drei wechselnden Werten von unterschiedlicher Herkunft und
Mitteln zur Anzeige der Werte, welche es erlauben die erzielten Werte qualitativ zusammen auf dem Bildschirm in einem einzelnen Grafikelement anzuzeigen,
wobei das Grafikelement eine bildhafte Darstellung einer Lungenform inkludiert und ein gegenwärtiges Abbild der Lungenform qualitative Informationen zur Compliance der Lunge beinhaltet,
**dadurch gekennzeichnet, dass** die Mittel zur Anzeige der Werte derart ausgebildet sind, dass eine Volumenveränderung der beatmeten Lunge, welche mit jedem Atemzug erzielt wird, in einer animierten Art durch eine Grössenänderung der Lungenform repräsentiert ist, welche mit der Volumenänderung korespondiert und eine Animation einer Konturlinie der Lungenform beinhaltet, deren Design qualitative Informationen zur Compliance der Lunge beinhalten.

2. Vorrichtung nach Anspruch 1, **dadurch gekennzeichnet, dass** ein Sensor zur Überwachung des Drucks und/oder des Flusses der Beatmungsluft vorgesehen ist, um eine Abschaltung oder eine Okklusion zu bestimmen und dass in beiden Fällen die Lungenform auf dem Bildschirm in konstanter Grösse angezeigt ist, wobei das konstante Ausmass der Lungenform im Fall dass eine Okklusion festgestellt wird, gross ist und das konstante Ausmass der Lungenform im Fall einer Abschaltung klein ist.

3. Vorrichtung nach Anspruch 1 oder 2, **dadurch gekennzeichnet, dass** die Änderung der Lungenform, welcher das Atmen repräsentiert, im Verhältnis zum gemessenen Volumenstrom steht.

4. Vorrichtung nach einem der Ansprüche 1 bis 4, **dadurch gekennzeichnet, dass** eine Bereichsteilung der Fläche der Lungenform vorgesehen ist, welche einen Unterschied zwischen einem idealen Lungenvolumen und einem beatmeten Lungenvolumen darstellt.

5. Vorrichtung nach einem der Ansprüche 1 bis 4, **dadurch gekennzeichnet, dass** ein ideales Lungenvolumen und/oder eine ideale Compliance durch eine Linie dargestellt ist, welche der Konturlinie der idealen Lungenform folgt oder einer Fläche mit der idealen Lungenform folgt, welche im Bezug auf Farbe und Helligkeit abgestuft ist.

6. Vorrichtung nach einem der Ansprüche 1 bis 5, **dadurch gekennzeichnet, dass** die angezeigte Lungenform auch eine Form des Atemwegs beinhaltet und ein momentaner Beatmungswiderstand durch die momentane Breite der Form des Luftwegs und/ oder durch den Farb- und Helligkeits-Bereich der Form des Luftwegs repräsentiert ist.

7. Vorrichtung nach einem der Ansprüche 1 bis 6, **dadurch gekennzeichnet, dass** das Grafikelement auch eine Form eines Muskelsystems beinhaltet, welches ein Muskelsystem , insbesondere ein Diaphragma, darstellt und wenigstens ein wechselnder Wert, welcher die Teilnahme des Patienten an der Atemleistung betrifft, mit der Form des Muskelsystems repräsentiert wird.

8. Vorrichtung nach einem der Ansprüche 1 bis 7, **dadurch gekennzeichnet, dass** das Grafikelement auch eine Gefässform und/oder eine Herzform beinhaltet und wenigstens ein wechselnder Wert, welcher die Blutzirkulation betrifft, mit der Gefässform und/oder der Herzform repräsentiert wird.

9. Vorrichtung nach Anspruch 8, **dadurch gekennzeichnet, dass** der momentane Blutdruck, insbesondere der momentane durchschnittliche Blutdruck, durch die momentane Breite der Gefässform und/oder der Herzform repräsentiert wird.

10. Vorrichtung nach einem der Ansprüche 8 oder 9, **dadurch gekennzeichnet, dass** eine momentane Sauerstoffsättigung des Bluts durch die momentane Farbe wenigstens eines Bereichs der Gefässform und/ oder der Herzform repräsentiert wird.

11. Vorrichtung nach einem der Ansprüche 8 bis 10, **dadurch gekennzeichnet, dass** eine Pulsanzeige, welche mit der Gefässform und/oder der Herzform mitläuft oder blinkt, angezeigt wird.

12. Vorrichtung nach einem der vorigen Ansprüche, **dadurch gekennzeichnet, dass** ein zweites Grafikelement auf dem Bildschirm angezeigt wird, welches wenigstens drei, bevorzugt mehr als drei und besonders bevorzugt alle der folgenden Parameter anzeigt:
- die angewendete Sauerstoffkonzentration "FiO₂'
- einen positiven endexspiratorischen Druck "PEEP"
- den inspiratorischen Druck "Pᵢₙₛₚ" oder den inspiratorischen Durchschnittsdruck "Pᵢₘₑₐₙ"
- das Verhältnis zwischen der kontrollierten Beatmung und dem spontanen Atmen "RR_{cont}/RRₛₚₒₙₜ" oder der schnellen flachen Atmung "RSB"
- das Verhältnis des theoretischen Minuten-Volumen (z.B. vom IBW abgeleitet) zum momentanen Minuten-Volumen "MV"
- den Varibilitätsindex und/oder
- einen Index für die Atemanstrengung des Patienten

13. Vorrichtung nach Anspruch 12, **dadurch gekennzeichnet, dass** die Werte der Parameter in einer analogen oder quasi-analogen Weise mit einer Position einer Teilungskomponente oder einer die Position wechselnden Komponente in den den Parametern zugewiesenen Massskalen angezeigt werden.

14. Vorrichtung nach einem der Ansprüche 12 oder 13, **dadurch gekennzeichnet, dass** der momentane Wert jedes repräsentierten Parameters im jeweiligen Fall in einem Bereich angezeigt wird, welche Bereiche im jeweiligen Fall an einem Ende schlechte Werte des entsprechenden Parameters anzeigen und gute Werte in einem Abstand dazu anzeigen und diese Bereiche so angeordnet sind, dass die guten Werte von allen Bereichen grafisch in Relation zueinander gesetzt sind.

15. Vorrichtung nach einem der Ansprüche 12 bis 14, **dadurch gekennzeichnet, dass** wenigsten ein Bereich mit guten Werten, ein Bereich mit schlechten Werten und, wenn es der jeweilige Fall erlaubt, ein Bereich mit mittleren Werten angezeigt wird und die Bereiche sich durch ihre Farbe, ihre Helligkeit und/oder durch ihre Musterung unterscheiden, wobei durch die Farbe, die Helligkeit und/ oder die Musterung angezeigt wird, ob alle angezeigten Werte im Bereich der guten Werte liegen.

## Revendications

1. Dispositif avec un écran pour représenter sur celui-ci des valeurs variables acquises avec la ventilation mécanique d'un patient,
avec des moyens pour acquérir au moins trois valeurs variables de différentes origines et
des moyens pour représenter les valeurs qui permettent aux valeurs acquises d'être représentées qualitativement ensemble sur l'écran en un seul élément graphique,
ledit élément graphique comprenant une représentation figurative de la forme d'un poumon, un dessin courant de la forme d'un poumon qui contient des informations qualitatives sur la compliance du poumon,
**caractérisé en ce que** les moyens pour représenter les valeurs sont conçus de telle manière qu'une variation de volume du poumon ventilé qui est acquise avec chaque respiration est représentée de manière animée par un changement de taille de la forme du poumon qui correspond à ce changement de volume en impliquant l'animation d'une ligne de contour de la forme du poumon dont le motif contient des informations qualitatives sur la compliance du poumon.

2. Dispositif selon la revendication 1, **caractérisé en ce qu'**un capteur pour surveiller la pression et/ou le flux de l'air de ventilation est présent pour être capable de constater un débranchement ou une occlusion et que dans les deux cas la forme du poumon est représentée avec une taille constante sur l'écran, la taille constante de la forme du poumon étant grande dans le cas de la constatation d'une occlusion et étant petite dans le cas de la constatation d'un débranchement.

3. Dispositif selon la revendication 1 ou 2, **caractérisé en ce que** le changement de zone de la forme du poumon qui représente la respiration est en relation avec le volume courant mesuré.

4. Dispositif selon l'une des revendications 1 à 4, **caractérisé en ce qu'**une division de zone de la zone de la forme du poumon est présente qui représente une différence entre un volume de poumon idéal et un volume de poumon ventilé.

5. Dispositif selon l'une des revendications 1 à 4, **caractérisé en ce qu'**un volume de poumon idéal et/ou une compliance idéale est représentée comme une ligne suivant la ligne de contour de la forme de poumon idéal ou une zone avec la forme du poumon idéal qui est dégradée en couleur ou en luminosité.

6. Dispositif selon l'une des revendications 1 à 5, **caractérisé en ce que** la forme du poumon représentée comprend aussi une forme des voies respiratoires et une résistance de ventilation courante est représentée au moyen de la largeur courante des voies respiratoires et/ou par la couleur ou le degré de luminosité de la forme des voies respiratoires.

7. Dispositif selon l'une des revendications 1 à 6, **caractérisé en ce que** l'élément graphique comprend aussi une forme de système de muscle qui représente un système de muscle, en particulier un diaphragme, et au moins une valeur variable concernant la participation du patient au travail respiratoire est représentée avec la forme du muscle,

8. Dispositif selon l'une des revendications 1 à 7, **caractérisé en ce que** l'élément graphique comprend aussi une forme de vaisseau et/ou une forme de coeur et au moins une valeur variable concernant la circulation du sang est représentée avec la forme du vaisseau et/ou la forme du coeur.

9. Dispositif selon la revendication 8, **caractérisé en ce qu'**une tension courante, en particulier la tension moyenne courante, est représentée par la largeur courante de la forme du vaisseau et/ou de la forme du coeur.

10. Dispositif selon l'une des revendications 8 ou 9, **caractérisé en ce qu'**une saturation en oxygène courante du sang est représentée au moyen de la couleur courante d'au moins une région de la forme du vaisseau et/ou de la forme du coeur.

11. Dispositif selon les revendications 8 à 10, **caractérisé en ce qu'**un symbole du pouls qui court le long d'une forme de vaisseau et/ou de la forme du coeur ou qui clignote est affiché.

12. Dispositif selon l'une des précédentes revendications, **caractérisé en ce qu'**un second élément graphique est représenté sur l'écran qui représente graphiquement au moins trois, de préférence plus que trois, de manière particulièrement préférée tous les paramètres suivants :
- la concentration en oxygène appliquée "FiO₂",
- une pression positive de fin d'expiration "PEEP",
- la pression inspiratoire "Pᵢₙₛₚ" ou la pression inspiratoire moyenne "Pᵢₘₑₐₙ",
- le rapport entre la ventilation contrôlée et la respiration spontanée "RR_{contr}./RRₛₚₒₙₜ"
ou la polypnée superficielle "RSB",
- le taux de volume minute théorique (par ex. dérivé de IBW) et du volume minute courant "MV",
- le taux de variabilité et/ou
- un indice d'effort respiratoire du patient.

13. Dispositif selon la revendication 12, **caractérisé en ce que** les valeurs de paramètres sont affichées de manière analogique ou quasi-analogique avec une position de division ou d'un composant changeant de position sur des échelles allouées aux paramètres.

14. Dispositif selon la revendication 12 ou 13, **caractérisé en ce que** la valeur courante de chacun des paramètres représentés est représentée dans chaque cas sur une plage, lesdites plages dans chaque cas affichant des valeurs faibles du paramètre respectif à une extrémité et des bonnes valeurs à une distance de celles-ci et ces valeurs sont arrangées de telle manière que les bonnes valeurs de toutes les plages sont mises en relation graphiquement les unes par rapport aux autres.

15. Dispositif selon l'une des revendications 12 à 14, **caractérisé en ce qu'**au moins une région avec des bonnes valeurs, une région avec des valeurs faibles et, le cas échéant, une région avec des valeurs moyennes sont représentées et sont différentes par leur couleur, leur luminosité et/ou par leurs motifs, dans lequel il est affiché par la couleur, la luminosité ou les motifs d'une région de chaque plage et/ou de l'arrière-plan de manière à voir si toutes les valeurs lues affichées se trouvent à l'intérieur de leur région avec de bonnes valeurs.
